# EUROPÄISCHE PATENTANMELDUNG

(11) **EP 4 585 926 A1**
(43) Veröffentlichungstag der Anmeldung: **16.07.2025**
(21) Anmeldenummer: 24150860.5
(22) Anmeldetag: 09.01.2024
(51) Int. Cl.: G01N 33/86

(54) **VERFAHREN UND KIT ZUR REDUZIERUNG VON INTERFERENZEN IN IMMUNOASSAYS**

(71) Anmelder: Siemens Healthcare Diagnostics Products GmbH, 35041 Marburg (DE)
(72) Erfinder: Patzke, Juergen, 35043 Marburg (DE)
(74) Vertreter: Siemens Healthineers Patent Attorneys

(57) **Zusammenfassung**

Die vorliegende Erfindung liegt auf dem Gebiet der Immunoassays für in vitro-diagnostische Anwendungen und betrifft die Verwendung eines testspezifischen blockierenden Antikörpers zur Reduzierung von Interferenzen, wie sie durch heterophile Antikörper oder Rheumafaktoren verursacht werden.

## Beschreibung

Die vorliegende Erfindung liegt auf dem Gebiet der Immunoassays für in vitro-diagnostische Anwendungen und betrifft die Verwendung eines testspezifischen blockierenden Antikörpers zur Reduzierung von Interferenzen, wie sie durch heterophile Antikörper oder Rheumafaktoren verursacht werden.

Immunoassays werden seit vielen Jahrzehnten in der klinischen Diagnostik zum quantitativen oder qualitativen Nachweis verschiedenster Analyten in Körperflüssigkeitsproben angewandt. Die Antikörper, die zum direkten Nachweis eines Antigens oder zum indirekten Nachweis eines anderen Analyten eingesetzt werden, sind typischerweise monoklonale oder polyklonale tierische Antikörper, die aus immunisierten Tieren (z.B. Kaninchen, Maus, Schaf) oder biotechnologisch gewonnen werden. Falsche Testergebnisse, die durch interferierende Substanzen aus der Patientenprobe verursacht werden, können zu weitreichenden Fehldiagnosen führen. Ein bekanntes und relativ häufig auftretendes Problem sind interferierende Antikörper, die in der Probe eines Individuums intrinsisch enthalten sein können (Bolstad, N. et al., Heterophilic antibody interference in immunometric assays. Best Practice & Research Clinical Endocrinology & Metabolism (2013), 647-661). Ein Beispiel für interferierende Antikörper sind heterophile Antikörper, also Antikörper im Blut des Patienten, die gegen Antigene, insbesondere gegen Immunglobuline, einer anderen Spezies gerichtet sind, wie z.B. humane anti-Maus Antikörper (HAMAs). Ein anderes Beispiel für interferierende Antikörper sind die sogenannten Rheumafaktoren oder rheumatoide Faktoren, also humane Autoantikörper, die gegen den Fc-Teil des humanen Immunglobulins G gerichtet sind. Alle diese interferierenden Antikörper weisen typischerweise eine Affinität für tierische Antikörper auf und binden häufig an den Fc-Teil. Wird in einem Testsystem nun ein tierischer Antikörper als Teil der Nachweisreaktion zum Nachweis eines Analyten verwendet ("analytischer Antikörper"), kann es in Gegenwart von interferierenden Antikörpern aus der Patientenprobe zu einer Bindungsreaktion zwischen analytischem und interferierenden Antikörpern kommen, die entweder die Nachweisreaktion blockiert und ein falsch-negatives/falsch zu niedriges Ergebnis verursacht oder die Nachweisreaktion verstärkt und ein falsch-positives/falsch zu hohes Ergebnis verursacht.

Modernen Immunoassays werden daher zur Reduzierung derartiger Interferenzen standardmäßig blockierende Antikörper hinzugefügt. Typischerweise handelt es sich um Mischungen von zufällig ausgewählten, unspezifischen Antikörpern derselben Immunglobulinklasse und derselben Spezies wie des in dem Testsystem verwendeten analytischen Antikörpers. So kann z.B. in einem Testsystem, in dem ein monoklonaler Mausantikörper (z.B. Maus-IgG1) als analytischer Antikörper eingesetzt wird, zusätzlich eine Mischung irrelevanter, also im Testsystem nicht funktioneller Maus-IgG1 im Überschuss zugegeben werden. Dies bewirkt in den meisten Fällen, dass etwaige interferierende Antikörper aus der Patientenprobe an die irrelevanten Antikörper binden, wodurch die störende Bindung an den analytischen Antikörper verhindert oder zumindest minimiert wird. Derartige blockierende Agenzien sind kommerziell erhältlich, z.B. Heterophilic Blocking Reagent Reagenzien (HBR) von Scantibodies Laboratory, Inc. oder TRU Block Reagenzien von Meridian Bioscience, Inc. Eine Optimierung der blockierenden Wirkung solcher irrelevanter Antikörper kann durch die vorherige Aggregation irrelevanter Antikörper erzielt werden (US 2004/0018556 A1).

Trotz dieser Maßnahmen gibt es dennoch Fälle, in denen bestimmte Proben mit einem spezifischen Immunoassay-Testsystem nicht korrekt analysiert werden können, da die Blockierung mit den herkömmlichen blockierenden Reagenzien offenbar nicht wirksam genug ist. Bowyer A.E. et al. (Von Willebrand factor activity assay errors. Haemophilia (2016), 22, e74-e76) beschreiben Patientenproben, für die mit zwei unterschiedlichen Testsystemen für die Bestimmung der von Willebrand Faktor (VWF)-Aktivität, die beide u.a. auch monoklonale Mausantikörper einsetzen, trotz Zugabe von HAMA Blockern (zur Blockierung humaner anti-Maus Antikörper) wiederholt falsch zu hohe Ergebnisse ermittelt wurden.

Ein "Immunoassay" im Sinne der vorliegenden Erfindung ist ein Verfahren zum Nachweis eines Analyten in einer Probe, das die Verwendung mindestens eines Antigen-spezifischen Antikörpers umfasst. Bei dem Antigen-spezifischen Antikörper kann es sich, muss es sich aber nicht zwangsläufig, um einen Analyt-spezifischen Antikörper handeln.

Je nach Testaufbau kann der verwendete Antikörper unterschiedlichste Funktionen erfüllen. Er kann beispielsweise als Fängerantikörper oder als markierter Zweitantikörper für die direkte Bindung und den Nachweis des Analyten dienen; dazu muss es sich um einen Analyt-spezifischen Antikörper handeln. In einem anderen Fall kann der Antikörper beispielsweise dazu dienen einen Bindungspartner des nachzuweisenden Analyten auf einer Festphase zu immobilisieren; dazu muss es sich um einen Antikörper mit Spezifität für diesen Bindungspartner handeln. Es sind verschiedenste Immunoassay-Prinzipien bekannt (direkt, indirekt, kompetitiv, nicht-kompetitiv). Allen gemein ist, dass sie die Verwendung mindestens eines Antigen-spezifischen Antikörpers umfassen, der in dem gewählten Testsystem zum Nachweis des Analyten direkt oder indirekt an der Analyt-spezifischen Nachweisreaktion beteiligt ist.

Herkömmliche Immunoassays, die dahingehend optimiert sind, dass das Auftreten von Antikörper-induzierten Interferenzen minimiert ist, sind also Verfahren zum Nachweis eines Analyten in einer Körperflüssigkeitsprobe im Wesentlichen mit den Schritten:
i) Bereitstellen eines Reaktionsgemisches durch In-Kontakt-Bringen der Probe mit
   a) einem Antigen-spezifischen Antikörper, der spezifisch an das Antigen bindet und
   b) einem Gemisch unspezifischer, zufällig ausgewählter Antikörper, die die Bindung von in der Probe enthaltenen interferierenden Antikörper an den Antigen-spezifischen Antikörper erfahrungsgemäß blockieren und
ii) Messen einer Messgröße in dem Reaktionsgemisch, wobei die Messgröße von der Bildung eines Komplexes aus Antigen und dem ersten, Antigen-spezifischen Antikörper beeinflusst wird, und die mit der Menge des Analyten korreliert.

Da es trotz der Zugabe von blockierenden Antikörpern zu dem Reaktionsgemisch, immer wieder Proben gibt, die in bestimmten Immunoassays nicht korrekt analysiert werden können, da die Blockierung offenbar nicht wirksam genug ist, liegt der vorliegenden Erfindung die Aufgabe zugrunde, weitere Verfahren und Mittel für Immunoassays bereitzustellen, die die Zuverlässigkeit von Immunoassays steigern, indem sie etwaige Interferenzen wirksam reduzieren, die durch in einer Patientenprobe enthaltene interferierende Antikörper, wie heterophile Antikörper oder Rheumafaktoren, verursacht werden.

Die Aufgabe wird erfindungsgemäß dadurch gelöst, dass ein von dem analytischen Antikörper abgeleiteter, blockierender Antikörper eingesetzt wird, der eine nahezu identische Struktur wie der analytische Antikörper aufweist. Dies bewirkt, dass etwaige interferierende Antikörper aus der Patientenprobe, die offenbar sehr spezifisch an ein Epitop des analytischen Antikörpers binden und daher durch die klassischen, zufällig ausgewählten blockierenden Antikörpermischungen nicht oder nicht ausreichend blockiert werden, durch Bindung an den abgeleiteten, blockierenden Antikörper nun gezielt abgefangen und damit blockiert werden. Es wurde gefunden, dass ein Antikörper mit einer Aminosäuresequenz, die mit Ausnahme von ein bis drei veränderten Aminosäureresten identisch ist mit der Aminosäuresequenz des analytischen Antikörpers, wodurch dessen Antigen-Bindungsfähigkeit im Vergleich zu dem analytischen Antikörper stark vermindert ist, eine effiziente Blockierung von interferierenden Antikörpern bewirkt und damit die Zuverlässigkeit des Immunoassays wesentlich verbessert.

Gegenstand der vorliegenden Erfindung ist daher ein Kit zur Verwendung in einem Verfahren zum Nachweis eines Analyten in einer Körperflüssigkeitsprobe. Das Kit enthält
i) einen ersten, Antigen-spezifischen Antikörper mit einer ersten Aminosäuresequenz, der spezifisch an ein Antigen bindet und dessen Verwendung in einem definierten Testsystem zum Nachweis des Analyten eine Analyt-spezifische Nachweisreaktion bewirkt ("analytischer Antikörper"), und
ii) eine Antikörpervariante mit einer zweiten Aminosäuresequenz, wobei die Antigen-Bindungsfähigkeit der Antikörpervariante im Vergleich zu dem ersten Antikörper so stark vermindert ist, beziehungsweise dass deren Kompetition mit dem ersten, Antigen-spezifischen Antikörper um die Bindung an das Antigen so gering ist, dass deren zusätzliche Verwendung in dem definierten Testsystem zum Nachweis des Analyten die Analyt-spezifische Nachweisreaktion um maximal 15 % verringert (auch "nicht-analytischer Antikörper" oder "nicht-analytische Antikörpervariante" genannt),
und wobei die Aminosäuresequenz der Antikörpervariante mit Ausnahme von ein bis drei veränderten Aminosäureresten identisch ist mit der Aminosäuresequenz des ersten, Antigen-spezifischen Antikörpers.

Ein Kit zur Verwendung in einem Verfahren zum Nachweis eines Analyten in einer Körperflüssigkeitsprobe umfasst typischerweise ein oder mehrere Reagenzien in flüssiger oder lyophilisierter Form oder in Form von beschichteten Festphasen, die mit der zu analysierenden Körperflüssigkeitsprobe (z.B. Vollblut, Plasma, Serum, Urin) in Kontakt gebracht werden, um eine Nachweisreaktion zu bewirken, die die quantitative, semi-quantitative oder qualitative Bestimmung der Menge oder der Aktivität des Analyten ermöglicht.

Der erste, Antigen-spezifische Antikörper bindet spezifisch an ein Antigen und ist unerlässlich für die Generierung der mit dem Testsystem beabsichtigten Analyt-spezifischen Nachweisreaktion ("analytischer Antikörper"). Bei dem Antigen-spezifischen Antikörper kann es sich um einen Analyt-spezifischen Antikörper handeln, der spezifisch an einen Analyten aus der Körperflüssigkeitsprobe bindet. Alternativ kann es sich um einen Antikörper handeln, der spezifisch an einen Bindungspartner des Analyten bindet. In diesem Fall kann es sich um einen Bindungspartner des Analyten handeln, der intrinsisch in der Körperflüssigkeitsprobe enthalten ist oder der dem Reaktionsgemisch zugegeben wird. In einer anderen Ausführungsform eines Nachweisverfahrens kann es sich um einen Antikörper handeln, der ein Spaltprodukt des Analyten spezifisch bindet.

Je nach Testaufbau kann der verwendete Antikörper unterschiedlichste Funktionen erfüllen. Er kann beispielsweise als Fängerantikörper oder als markierter Zweitantikörper für die direkte Bindung und den Nachweis den Analyten dienen; dazu muss es sich um einen Analyt-spezifischen Antikörper handeln. In einem anderen Fall kann der Antikörper beispielsweise dazu dienen einen Bindungspartner des nachzuweisenden Analyten auf einer Festphase zu immobilisieren; dazu muss es sich um einen Antikörper mit Spezifität für diesen Bindungspartner handeln. Es sind verschiedenste Immunoassay-Prinzipien bekannt (direkt, indirekt, kompetitiv, nicht-kompetitiv). Allen gemein ist, dass sie die Verwendung mindestens eines Antigen-spezifischen Antikörpers umfassen, der in dem gewählten Testsystem zum Nachweis des Analyten direkt oder indirekt an der Analyt-spezifischen Nachweisreaktion beteiligt ist.

Der erste, Antigen-spezifische Antikörper kann einer beliebigen Immunglobulinklasse angehören (IgA, IgD, IgE, IgG oder IgM); er kann ursprünglich aus Mensch, Maus, Kaninchen, Maus, Schaf, Kamel oder einem anderen Tier stammen. Bevorzugterweise handelt es sich um einen monoklonalen oder um einen rekombinant hergestellten Antikörper. Der erste, Antigen-spezifische Antikörper kann auch ein chimärer oder ein humanisierter Antikörper sein. Der Begriff "erster, Antigen-spezifischer Antikörper" umfasst ausdrücklich nicht nur komplette Antikörper, sondern auch verschiedene Antigenbindende Antikörperfragmente, wie z.B. Fab- oder F(ab)₂-Fragmente.

In verschiedenen Ausführungsformen kann der erste, Antigen-spezifische Antikörper mit einer Festphase und/oder einer Komponente eines signalgebenden Systems assoziiert sein.

Der Begriff "Festphase" im Sinne dieser Erfindung beinhaltet einen Gegenstand, der aus porösem und/oder nicht porösem, wasserunlöslichem Material besteht und die unterschiedlichsten Formen aufweisen kann, wie z. B. Gefäß, Röhrchen, Mikrotitrationsplatte (ELISA-Platte), Kugel, Mikropartikel, Stäbchen, Streifen, Filter- oder Chromatographiepapier, etc. In der Regel ist die Oberfläche der Festphase hydrophil oder kann hydrophil gemacht werden. Die Festphase kann aus den unterschiedlichsten Materialien bestehen wie z. B. aus anorganischen und/oder aus organischen Materialien, aus synthetischen, aus natürlich vorkommenden und/oder aus modifizierten natürlich vorkommenden Materialien. Beispiele für Festphasenmaterialien sind Polymere wie z. B. Cellulose, Nitrocellulose, Zelluloseacetat, Polyvinylchlorid, Polyacrylamid, vernetzte Dextranmoleküle, Agarose, Polystyrol, Polyethylen, Polypropylen, Polymethacrylat oder Nylon; Latex; Keramik; Glas; Metalle, insbesondere Edelmetalle wie Gold und Silber; Magnetit; Mischungen oder Kombinationen derselben. Partikel, einschließlich magnetische Partikel und Latexpartikel, können mit Farbstoffen, Sensitizern, fluoreszierenden Substanzen, chemilumineszierenden Substanzen, Isotopen oder anderen nachweisbaren Labeln markiert sein.

Bei einer "Komponente eines signalbildenden Systems" handelt es sich um ein Molekül, das selbst ein Signal produziert oder die Produktion eines Signals induzieren kann, wie z.B. eine fluoreszierende Substanz, eine chemilumineszierende Substanz eine radioaktive Substanz oder ein Enzym. Das Signal kann beispielsweise anhand der Enzymaktivität, der Lumineszenz, der Lichtabsorption, der Lichtstreuung, der ausgestrahlten elektromagnetischen oder radioaktiven Strahlung oder einer chemischen Reaktion nachgewiesen oder gemessen werden.

Geeignete Komponenten eines signalbildenden Systems sind beispielsweise Enzyme, einschließlich Meerrettichperoxidase, alkalische Phosphatase, Glukose-6-Phosphatdehydrogenase, Alkoholdehydrogenase, Glucoseoxidase, β-Galactosidase, Luciferase, Urease und Acetylcholinesterase; Enzymsubstrate; Farbstoffe; fluoreszierende Substanzen, einschließlich Fluoresceinisothiocyanat, Rhodamin, Phycoerythrin, Phycocyanin, Ethidiumbromid, 5-Dimethylaminonapthalen-1-sulfonylchlorid und fluoreszierende Chelate von seltenen Erden; chemilumineszierende Substanzen einschließlich Luminol, Isoluminol, Acridiniumverbindungen, Olefin, Enolether, Enamin, Arylvinylether, Dioxen, Arylimidazol, Lucigenin, Luciferin und Aequorin; Sensitizer einschließlich Eosin, 9,10-Dibromoanthracen, Methylen Blau, Porphyrin, Phthalocyanin, Chlorophyll, Rose Bengal; Coenzyme; radioaktive Isotope einschließlich ¹²⁵I, ¹³¹I, ¹⁴C, ³H, ³²P, ³³P, ³⁵S, ⁵¹Cr, ⁵⁹Fe, ⁵⁷Co und ⁷⁵Se.

Der Begriff "assoziiert" ist breit zu verstehen und umfasst beispielsweise eine kovalente und eine nicht-kovalente Bindung, eine direkte und eine indirekte Bindung, die Adsorption an eine Oberfläche und den Einschluss in eine Vertiefung. Bei einer kovalenten Bindung ist der erste, Antigen-spezifische Antikörper bzw. das Antigen-spezifische Antikörperfragment über eine chemische Bindung an eine Festphase oder an eine Komponente eines signalbildenden Systems gebunden. Ein Beispiel für eine nicht-kovalente Bindung ist die Oberflächenadsorption. Neben einer direkten Bindung kann der erste, Antigen-spezifische Antikörper bzw. das Antigen-spezifische Antikörperfragment auch indirekt über spezifische Wechselwirkung mit anderen Bindungspartnern an die Festphase gebunden sein, z.B. über spezifische Wechselwirkung mit Avidin, sofern der erste, Antigen-spezifische Antikörper bzw. das Antigen-spezifische Antikörperfragment biotinyliert ist.

Die Antikörpervariante des erfindungsgemäßen Kits ist ein vom dem ersten, Antigen-spezifischen Antikörper abgeleiteter Antikörper beziehungsweise ein entsprechendes Antikörperfragment (auch "nicht-analytischer Antikörper" oder "nicht-analytische Antikörpervariante" genannt) und weist 1.) eine Aminosäuresequenz auf, die mit Ausnahme von ein bis drei veränderten Aminosäureresten identisch ist mit der Aminosäuresequenz des ersten, Antigen-spezifischen Antikörpers und 2.) eine Antigen-Bindungsfähigkeit, die im Vergleich zu dem ersten, Antigen-spezifischen Antikörper so stark vermindert ist, dass ihre zusätzliche Verwendung in dem definierten Testsystem zum Nachweis des Analyten die Analyt-spezifische Nachweisreaktion um maximal 15 % verringert.

Eine solche "nicht-analytische" Antikörpervariante ist also individuell auf den ersten, Antigen-spezifischen Antikörper ("analytischer Antikörper") abgestimmt und ist typischerweise dadurch erhältlich, dass ausgehend von der bekannten Aminosäuresequenz des analytischen Antikörpers (oder nach Ermittlung derselben) durch den Austausch, die Deletion, die Insertion oder die chemische Derivatisierung von ein bis drei Aminosäureresten eine veränderte Aminosäuresequenz festgelegt wird, und eine entsprechend modifizierte Antikörpervariante rekombinant hergestellt wird. Die Position des einen veränderten Aminosäurerests bzw. die Positionen der zwei oder drei veränderten Aminosäurereste sind so zu wählen, dass sie in einem Bereich des analytischen Antikörpers liegen, der für die Antigenbindung relevant ist und der durch die Veränderung der Aminosäuresequenz funktionell eingeschränkt bzw. ausgeschaltet werden soll, so dass die resultierende veränderte, nicht-analytische Antikörpervariante keine oder zumindest nur noch eine stark verminderte Antigen-Bindungsfähigkeit aufweist. Zu diesem Zweck werden die ein bis drei Aminosäurereste vorzugsweise in einer oder mehreren der komplementaritätsbestimmenden Regionen (CDR's) der schweren oder der leichten Kette des analytischen Antikörpers modifiziert, also ausgetauscht, deletiert, insertiert oder chemisch derivatisiert. Die komplementaritätsbestimmenden Regionen (CDR's) der schweren Kette eines Antikörpers (CDR-H1, CDR-H2 und CDR-H3) und der leichten Kette eines Antikörpers (CDR-L1, CDR-L2 und CDR-L3) (gemäß Kabat-Nummerierungsschema), welche durch die sogenannten Framework-Regionen voneinander getrennt sind, sind dem Fachmann hinreichend bekannt. Besonders bevorzugt wird mindestens ein Aminosäurerest in einer komplementaritätsbestimmenden Region der schweren Kette des analytischen Antikörpers modifiziert. Weiter bevorzugt wird mindestens ein Aminosäurerest in der komplementaritätsbestimmenden Region CDR-H3 der schweren Kette des analytischen Antikörpers modifiziert.

In einer Ausführungsform des Testkits ist also eine Antikörpervariante enthalten, bei der die ein bis drei veränderten Aminosäurereste in einer oder mehreren der komplementaritätsbestimmenden Regionen (CDR's) der schweren oder der leichten Kette der Antikörpervariante lokalisiert sind.

In einer weiteren Ausführungsform des Testkits ist eine Antikörpervariante enthalten, bei der mindestens ein veränderter Aminosäurerest in einer komplementaritätsbestimmenden Region der schweren Kette der Antikörpervariante lokalisiert ist.

In noch einer weiteren Ausführungsform des Testkits ist eine Antikörpervariante enthalten, bei der mindestens ein veränderter Aminosäurerest in der komplementaritätsbestimmenden Region CDR-H3 der schweren Kette der Antikörpervariante lokalisiert ist.

Unter einem "veränderten Aminosäurerest" ist ein Aminosäurerest zu verstehen, der in Bezug auf eine Position in der Aminosäurefolge der Primärsequenz des analytischen Antikörpers ausgetauscht, deletiert, insertiert oder chemisch derivatisiert ist. Bei einem Austausch (Substitution) wird der ursprüngliche Aminosäurerest durch einen anderen Aminosäurerest ersetzt. Vorzugsweise sind die Substitutionen nicht-konservative Substitutionen, also solche die zwischen unterschiedlichen Familien von Aminosäuren stattfinden, die sich in ihren Seitenketten und chemischen Eigenschaften unterscheiden. Beispiele für unterschiedliche Familien sind Aminosäuren mit basischen Seitenketten, mit sauren Seitenketten, mit unpolaren aliphatischen Seitenketten, mit unpolaren aromatischen Seitenketten, mit polaren Seitenketten, mit ungeladenen polaren Seitenketten, mit geladenen Seitenketten, mit kleinen Seitenketten, mit großen Seitenketten usw. Beispielsweise wird ein kleiner durch einen großen Aminosäurerest ersetzt oder ein geladener wird durch einen ungeladenen Aminosäurerest ersetzt.

Die "nicht-analytische" Antikörpervariante kann auch eine natürlich vorkommende Variante des ersten, Antigen-spezifischen Antikörpers sein, wobei die Aminosäuresequenz der Variante ein bis drei ausgetauschte, deletierte oder insertierte Aminosäurereste gegenüber dem ursprünglichen analytischen Antikörper aufweist.

Die Antikörpervariante ist also verglichen mit dem ersten, Antigen-spezifischen Antikörper eine in Bezug auf die Antigen-Bindungsfähigkeit nicht-funktionelle oder zumindest minder-funktionelle Variante davon.

Die Antigen-Bindungsfähigkeit der Antikörpervariante muss im Vergleich zu dem ersten, Antigen-spezifischen Antikörper so stark vermindert sein, dass deren zusätzliche Verwendung in dem definierten Testsystem zum Nachweis des Analyten die Analyt-spezifische Nachweisreaktion um maximal 15 % verringert.

Eine verminderte Antigen-Bindungsfähigkeit der Antikörpervariante kann in einem vergleichenden Experiment mit dem ersten, Antigen-spezifischen Antikörper in einem Standard-Assay zur Bestimmung der Spezifität der Bindung des Zielantigens gemessen werden, beispielsweise in einem ELISA-Assay, BIAcore-Assay, Octet BLI-Assay oder in einem FACSbasierten Assay, wenn das Antigen auf einer Zelloberfläche exprimiert wird.

Ausschlaggebend ist jedoch, dass die Antigen-Bindungsfähigkeit der Antikörpervariante in demselben definierten Testsystem zum Nachweis des Analyten getestet wird, in dem der erste, Antigen-spezifische Antikörper als "analytischer" Antikörper zum Einsatz kommt. Der Begriff "definiertes Testsystem" bezeichnet einen Testaufbau, der hinsichtlich der eingesetzten Komponenten und der Verfahrensschritte festgelegt ist. Die Variation einer einzelnen Komponente oder eines einzelnen Verfahrensschrittes in dem ansonsten unveränderten Testaufbau ermöglicht es, den Einfluss dieser Variation in dem ansonsten definierten Testsystem zu ermitteln. Idealerweise wird das Verfahren zum Nachweis eines Analyten als definiertes Testsystem verwendet, für dessen Durchführung die Verwendung des ersten, Antigen-spezifischen Antikörpers und der Antikörpervariante vorgesehen ist. In Bezug auf die Erfindung werden dazu der erste, Antigen-spezifische Antikörper zwecks Nachweis des Analyten und zusätzlich, das heißt in Kombination mit dem ersten, Antigen-spezifische Antikörper, die zu testende Antikörpervariante eingesetzt.

Eine geeignete Antikörpervariante hat in dem Testsystem, in dem der erste, Antigen-spezifische Antikörper als "analytischer" Antikörper eingesetzt wird, keinen wesentlichen kompetitiven Effekt auf die Analyt-spezifische Nachweisreaktion. Um dies sicherzustellen, wird vorab in dem Testsystem über den gesamten Messbereich die Reaktionsstärke der Analyt-spezifischen Nachweisreaktion mit und ohne Zugabe der Antikörpervariante zum Reaktionsgemisch in Proben ohne interferierende Antikörper gemessen. Eine geeignete Antikörpervariante ist solch eine, deren Antigen-Bindungsfähigkeit im Vergleich zu dem ersten, Antigen-spezifischen Antikörper so stark vermindert ist, dass ihre Anwesenheit die Reaktionsstärke der Analyt-spezifischen Nachweisreaktion nicht um mehr als 15 %, bevorzugterweise nicht um mehr als 10 %, besonders bevorzugt nicht um mehr als 5 % verringert; die verminderte Antigen-Bindungsfähigkeit der Antikörpervariante wird durch die geringfügige bis fehlende Kompetition der Antikörpervariante mit dem ersten, Antigen-spezifischen Antikörper um die Bindung an das Antigen, also die geringfügige bis fehlende Konkurrenz um die Bindungsstelle(n) des Antigens, funktionell nachgewiesen, indem gezeigt wird, dass die Verwendung der Antikörpervariante in Kombination mit dem ersten Antigen-spezifischen Antikörper keinen übermäßig störenden Einfluss auf die Analyt-spezifische Nachweisreaktion hat, sondern eine maximale Verringerung der Reaktionsstärke der Analyt-spezifischen Nachweisreaktion von 15 % bewirkt.

In einem erfindungsgemäßen Kit können der erste, Antigen-spezifische Antikörper und die Antikörpervariante in unterschiedlichen Reagenzien oder in einem einzigen Reagenz enthalten sein.

In einer Ausführungsform des Kits ist der erste, Antigen-spezifische Antikörper mit einer Festphase, wie z.B auf der Oberfläche eines Gefäßes assoziiert (wie weiter oben beschrieben), beispielsweise auf dem Boden einer Kavität einer Mikrotiterplatte oder auf der Innenseite eines Reaktionsgefäßes. Ein solches Kit eignet sich besonders für die Durchführung heterogener Testverfahren, wie z.B. ELISA-Teste. Ein solches Kit enthält vorzugsweise ferner ein weiteres Gefäß, das die Antikörpervariante enthält, vorzugsweise als Bestandteil eines flüssigen Reagenzes (oder eines Lyophilisats desselben).

In einer anderen Ausführungsform des Kits ist der erste, Antigen-spezifische Antikörper mit einer Oberfläche einer partikulären Festphase assoziiert (wie weiter oben beschrieben). Dazu enthält das Kit ein Gefäß, welches das entsprechende Reagenz in Form einer flüssigen Suspension oder eines resuspendierbaren Lyophilisats derselben enthält. Ein solches Testkit eignet sich für die Messung der Agglutination mittels photometrischer Methoden.

In noch einer anderen Ausführungsform des Kits ist der erste, Antigen-spezifische Antikörper mit einer Komponente eines signalbildenden Systems assoziiert (wie weiter oben beschrieben). Der erste, Antigen-spezifische Antikörper kann direkt mit einer Komponente eines signalbildenden Systems assoziiert sein oder indirekt, beispielsweise wenn der Antikörper und die Komponente eines signalbildenden Systems mit einer einzigen Festphase, z.B. mit einem Latexpartikel, assoziiert sind. Dazu enthält das Kit ein Gefäß, welches das entsprechende Reagenz in flüssiger Form oder als resuspendierbares Lyophilisat desselben enthält. Ein solches Testkit eignet sich je nach Art des signalbildenden Systems zum Beispiel für Messungen der Chemilumineszenz, Fluoreszenz oder Absorptionsänderung.

In einem besonders bevorzugten Kit ist der erste, Antigen-spezifische Antikörper ein Analyt-spezifischer Antikörper. In diesem Fall dient der Antikörper direkt als Fängerantikörper oder als markierter Zweitantikörper für die Bindung und den Nachweis des Analyten, wie beispielsweise in einem Sandwich-Immunoassay.

In einer anderen Ausführungsform des Kits ist neben dem ersten Antigen-spezifischen Antikörper zusätzlich ein anderer, zweiter Antigen-spezifischer Antikörper enthalten, wie beispielsweise in einem Kit zur Verwendung in einem Sandwich-Immunoassay, wobei der zweite Antigen-spezifische Antikörper für dasselbe Antigen spezifisch sein kann wie der erste Antigen-spezifische Antikörper oder für ein anderes Antigen. In einem solchen Kit ist vorzugweise eine weitere "nicht-analytische" Antikörpervariante enthalten (wie weiter oben beschrieben), die verglichen mit dem zweiten, Antigen-spezifischen Antikörper eine in Bezug auf die Antigen-Bindungsfähigkeit nicht-funktionelle oder zumindest minder-funktionelle Variante davon ist. Ein solches Kit enthält also zusätzlich
c) einen zweiten, Antigen-spezifischen Antikörper mit einer dritten Aminosäuresequenz der spezifisch an ein Antigen bindet und dessen Verwendung in einem definierten Testsystem zum Nachweis des Analyten eine Analyt-spezifische Nachweisreaktion bewirkt und
d) eine weitere Antikörpervariante mit einer vierten Aminosäuresequenz, deren Antigen-Bindungsfähigkeit im Vergleich zu dem zweiten, Antigen-spezifischen Antikörper so stark vermindert ist, dass deren zusätzliche Verwendung in dem definierten Testsystem zum Nachweis des Analyten die Analyt-spezifische Nachweisreaktion um maximal 15 % verringert,
und wobei die Aminosäuresequenz der weiteren Antikörpervariante mit Ausnahme von ein bis drei veränderten Aminosäureresten identisch ist mit der Aminosäuresequenz des zweiten, Antigen-spezifischen Antikörpers.

Vorzugweise ist in einem erfindungsgemäßen Kit für jeden enthaltenen Antigen-spezifischen Antikörper eine Antikörpervariante vorgesehen, die verglichen mit dem jeweiligen Antigen-spezifischen Antikörper eine in Bezug auf die Antigen-Bindungsfähigkeit nicht-funktionelle oder zumindest minder-funktionelle Variante (wie oben beschrieben) davon ist.

Ein weiterer Gegenstand der vorliegenden Erfindung ist die Verwendung eines erfindungsgemäßen Kits in einem Verfahren zum Nachweis eines Analyten in einer Körperflüssigkeitsprobe.

Besonders bevorzugt ist die Verwendung eines erfindungsgemäßen Kits zum interferenzfreien Nachweis eines Analyten in einer Körperflüssigkeitsprobe, die interferierende Antikörper, beispielsweise aus der Gruppe heterophile Antikörper und Autoantikörper, enthält.

In einer speziellen Ausführungsform des Kits ist der erste, Antigen-spezifische Antikörper ein Antikörper, der spezifisch an Glykoprotein Ib (GPIb) -Protein bindet. Das GPIb-Protein ist ein Bindungspartner des von Willebrand Faktors (VWF) und wird in verschiedenen Assays zur Bestimmung der VWF-Aktivität eingesetzt (siehe z.B. WO 2009/007051 A2). Qualitative Defekte bzw. Funktionsstörungen des VWF werden durch eine verminderte Bindung des in der Probe enthaltenen VWF an zugegebenes GPIb-Protein nachgewiesen. Um die Bindungsfähigkeit des VWF an zugegebenes GPIb-Protein quantitativ bestimmen zu können, sind die Testverfahren so aufgebaut, dass die Komplexbildung von VWF und GPIb im Testansatz gemessen werden kann, beispielsweise durch Messung der Agglutination von Latexpartikeln, die mit einem anti-GPIb-Antikörper beschichtet sind und die nur dann agglutinieren, wenn VWF-GPIb-Komplexe im Testansatz entstehen, die dann wiederum von den Latexpartikel-assoziierten anti-GPIb-Antikörpern gebunden werden. Wie weiter oben erwähnt, wurde beobachtet, dass ein solcher Assay, bei dem ein monoklonaler anti-GPIb-Antikörper aus der Maus eingesetzt wird, trotz Zugabe von HAMA Blockern (zur Blockierung humaner anti-Maus Antikörper) wiederholt falsch zu hohe Ergebnisse liefert.

In einer bevorzugten Form der speziellen Ausführungsform des Kits ist
- der erste, Antigen-spezifische Antikörper ein Antikörper, der spezifisch an Glykoprotein Ib (GPIb) - Protein bindet und der in der komplementaritätsbestimmenden Region CDR-H3 der schweren Kette (gemäß Kabat-Nummerierungsschema) die Aminosäuresequenz gemäß SEQ ID NO. 1 (DTMIKGHYVMDY) aufweist, und
- die Antikörpervariante ein Antikörper, dessen Aminosäuresequenz mit Ausnahme von zwei veränderten Aminosäureresten identisch ist mit der Aminosäuresequenz des ersten GPIb-Protein-spezifischen Antikörpers und der in der komplementaritätsbestimmenden Region CDR-H3 der schweren Kette (gemäß Kabat-Nummerierungsschema) die Aminosäuresequenz gemäß SEQ ID NO. 2 (DTMIKGHSVFDY) aufweist.

Ein solches Testkit eignet sich für die Verwendung in einem Verfahren zum Nachweis der VWF-Aktivität in einer Körperflüssigkeitsprobe und hat den besonderen Vorteil, dass es den interferenzfreien Nachweis der VWF-Aktivität in einer Körperflüssigkeitsprobe ermöglicht, die interferierende Antikörper, beispielsweise aus der Gruppe heterophile Antikörper und Autoantikörper, enthält.

Ein weiterer Gegenstand der vorliegenden Erfindung ist ein Verfahren zum Nachweis eines Analyten in einer Körperflüssigkeitsprobe, das Verfahren umfassend die Schritte:
a) Bereitstellen eines Reaktionsgemisches durch Vermischen der Probe mit
   i. einem ersten, Antigen-spezifischen Antikörper mit einer ersten Aminosäuresequenz, der spezifisch an ein Antigen bindet und dessen Verwendung in einem definierten Testsystem zum Nachweis des Analyten eine Analyt-spezifische Nachweisreaktion bewirkt, und
   ii. einer Antikörpervariante mit einer zweiten Aminosäuresequenz, dessen Antigen-Bindungsfähigkeit im Vergleich zu dem ersten Antikörper so stark vermindert ist, dass deren zusätzliche Verwendung in dem definierten Testsystem zum Nachweis des Analyten die Analyt-spezifische Nachweisreaktion um maximal 15 % verringert; und
b) Messen einer Messgröße in dem Reaktionsgemisch, wobei die Messgröße von der Bildung eines Komplexes aus Antigen und dem ersten, Antigen-spezifischen Antikörper beeinflusst wird, und die mit der Menge des Analyten korreliert,
wobei auch hier (wie bereits weiter oben beschrieben) die Aminosäuresequenz der Antikörpervariante mit Ausnahme von ein bis drei veränderten Aminosäureresten identisch ist mit der Aminosäuresequenz des ersten, Antigen-spezifischen Antikörpers.

Bevorzugterweise wird die Probe zuerst mit der Antikörpervariante vermischt, das so entstandene Gemisch wird inkubiert und dann erst wird dem Gemisch der erste, Antigen-spezifische Antikörper zugegeben. Durch diese Präinkubation der Probe mit der "nicht-analytischen" Antikörpervariante wird eine besonders effiziente Blockierung interferierender Antikörper bewirkt, weil sie bereits gebunden sind, bevor sie mit dem "analytischen" Antikörper überhaupt in Kontakt kommen.

In einer Ausführungsform des erfindungsgemäßen Verfahrens ist der erste, Antigen-spezifische Antikörper ein Analyt-spezifischer Antikörper und die gemessene Messgröße wird von der Bildung eines Komplexes aus Analyt und dem ersten, Analyt-spezifischen Antikörper beeinflusst. Ein Beispiel dafür ist ein Immunoassay, in dem ein Komplex aus Analyt und einem Latexpartikel-assoziierten Analyt-spezifischen Antikörper gebildet wird und die Komplexbildung anhand der Agglutinationsreaktion der Latexpartikel im Reaktionsgemisch photometrisch bestimmt wird.

In einer anderen Ausführungsform des erfindungsgemäßen Verfahrens ist der erste, Antigen-spezifische Antikörper ein Antikörper mit Spezifität für einen Bindungspartner des Analyten und die gemessene Messgröße wird von der Bildung eines Komplexes aus Analyt und dem Bindungspartner des Analyten und dem ersten, Antigen-spezifischen Antikörper mit Spezifität für den Bindungspartner des Analyten beeinflusst. Ein Beispiel dafür ist ein funktioneller Bindungstest, mit dem nicht die Menge, sondern vielmehr die Bindungsfähigkeit eines Analyten an einen bestimmten Bindungspartner gemessen werden soll und in dem ein Komplex aus Analyt, Bindungspartner des Analyten und einem beispielsweise Latexpartikel-assoziierten Antikörper mit Spezifität für den Bindungspartner gebildet wird und die Komplexbildung anhand der Agglutinationsreaktion der Latexpartikel im Reaktionsgemisch photometrisch bestimmt wird.

Eine spezielle Ausführungsform des erfindungsgemäßen Verfahrens ist ein Verfahren zum Nachweis der Aktivität des von Willebrand Faktors in einer Körperflüssigkeitsprobe, wobei der erste, Antigen-spezifische Antikörper ein Antikörper mit Spezifität für GPIb-Protein ist und wobei die gemessene Messgröße von der Bildung eines Komplexes aus von Willebrand Faktor und dem GPIb-Protein und dem ersten, Antigen-spezifischen Antikörper mit Spezifität für GPIb-Protein beeinflusst wird.

In dem erfindungsgemäßen Verfahren kann der erste, Antigen-spezifische Antikörper mit einer partikulären Festphase assoziiert sein und die Agglutination der partikulären Festphase, die von der Bildung eines Komplexes aus Antigen und dem ersten, Antigen-spezifischen Antikörper beeinflusst wird und die mit der Menge des Analyten korreliert, im Reaktionsgemisch gemessen werden.

Die Messung der Agglutination der partikulären Festphase im Reaktionsgemisch kann photometrisch erfolgen, beispielsweise turbidimetrisch oder nephelometrisch. Bindungsteste beruhend auf dem Prinzip der partikelverstärkten Lichtstreuung sind seit etwa 1920 bekannt (zur Übersicht siehe Newman, D.J. et al., Particle enhanced light scattering immunoassay. Ann Clin Biochem 1992; 29: 22-42). Bevorzugterweise werden in diesem Zusammenhang Polystyrolpartikel mit einem Durchmesser von 0,1 bis 0,5 um, besonders bevorzugt mit einem Durchmesser von 0,15 bis 0,35 um verwendet. Bevorzugt werden Polystyrolpartikel mit Amin-, Carboxyl- oder Aldehydfunktionen verwendet. Weiterhin bevorzugt werden Schale/Kern-Partikel verwendet. Die Synthese der Partikel und die kovalente Kopplung von Liganden ist z.B. in Peula, J.M. et al., Covalent coupling of antibodies to aldehyde groups on polymer carriers. Journal of Materials Science: Materials in Medicine 1995; 6: 779-785 beschrieben.

Alternativ kann die Messung der Agglutination der partikulären Festphase im Reaktionsgemisch durch die Messung eines Signals erfolgen, das von einem signalbildenden System erzeugt wird, wenn eine erste und eine zweite Komponente des signalbildenden Systems in räumliche Nähe zueinander gebracht werden. In diesem Zusammenhang ist eine erste Fraktion der partikulären Festphase mit einer ersten Komponente eines signalbildenden Systems assoziiert, und eine zweite Fraktion der partikulären Festphase ist mit einer zweiten Komponente des signalbildenden Systems assoziiert, wobei die erste und zweite Komponente des signalbildenden Systems so zusammenwirken, dass ein detektierbares Signal entsteht, wenn die erste und die zweite Komponente des signalbildenden Systems in räumliche Nähe zueinander gebracht werden, und die Agglutination der partikulären Festphase im Reaktionsgemisch anhand des entstandenen Signals gemessen wird.

In dieser Ausführungsform des erfindungsgemäßen Verfahrens umfasst das signalbildende System mindestens eine erste und eine zweite Komponente, welche so zusammenwirken, dass ein detektierbares Signal entsteht, wenn sie in räumliche Nähe zueinander gebracht werden und dadurch miteinander in Wechselwirkung treten können. Unter einer Wechselwirkung zwischen den Komponenten ist insbesondere ein Energietransfer - also die direkte Übertragung von Energie zwischen den Komponenten, z.B. durch Licht- oder Elektronenstrahlung sowie über reaktive chemische Moleküle, wie z.B. kurzlebigen Singulett-Sauerstoff - zu verstehen. Der Energietransfer kann von einer auf eine andere Komponente erfolgen, möglich ist aber auch eine Kaskade verschiedener Substanzen über die der Energietransfer läuft. Zum Beispiel kann es sich bei den Komponenten um ein Paar aus einem Energiespender und einem Energieempfänger handeln, wie beispielsweise Photosensitizer und chemilumineszierendes Agens (EP-A2-0515194, LOCI^{®} Technologie) oder Photosensitizer und Fluorophor (WO 95/06877) oder radioaktives Iod<125> und Fluorophor (Udenfriend et al. (1985) Proc. Natl. Acad. Sci. 82: 8672 - 8676) oder Fluorophor und Fluoreszenz-Quencher (US 3,996,345). Besonders bevorzugt ist die erste Komponente des signalbildenden Systems ein chemilumineszierendes Agens und die zweite Komponente des signalbildenden Systems ein Photosensitizer oder umgekehrt, und es wird die Chemilumineszenz im Reaktionsgemisch gemessen.

Die folgenden Beispiele und die Figuren dienen der Veranschaulichung der vorliegenden Erfindung und sind nicht als Einschränkung zu verstehen.

Unabhängig vom grammatikalischen Geschlecht eines bestimmten Begriffes sind Personen mit männlicher, weiblicher oder anderer Geschlechtsidentität mit umfasst.

### FIGURENBESCHREIBUNG

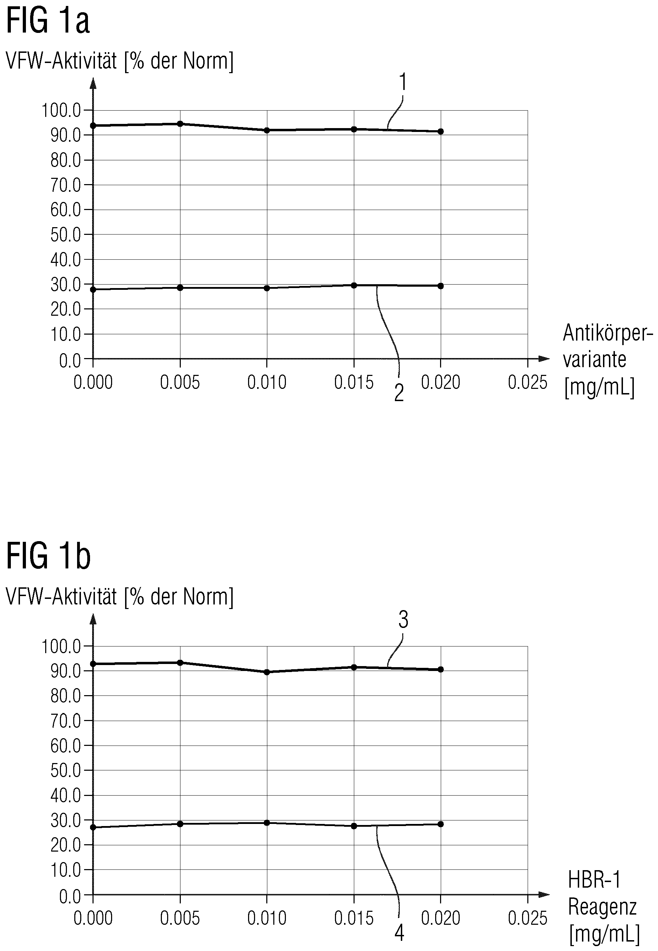

- FIG. 1A: zeigt ein Diagramm, in dem die in einer normalen Probe (1) und in einer Probe mit verminderter VWF-Aktivität (2) gemessenen VWF-Aktivitäten [% der Norm] in Abwesenheit und in Anwesenheit der neuen Antikörpervariante in unterschiedlichen Konzentrationen im Reaktionsgemisch (mg/mL) dargestellt sind.
- FIG. 1B: zeigt ein Diagramm, in dem die in einer normalen Probe (3) und in einer Probe mit verminderter VWF-Aktivität (4) gemessenen VWF-Aktivitäten [% der Norm] bei Verwendung verschiedener Verdünnungen des kommerziellen HBR-1-Reagenzes (mg/mL Gesamtproteingehalt im Reaktionsgemisch) dargestellt sind.

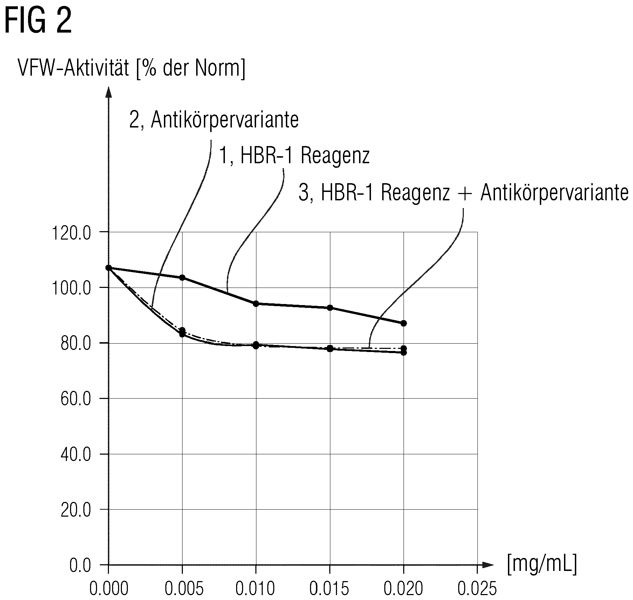

- FIG. 2: zeigt ein Diagramm, in dem die in einer HAMA-Antikörper enthaltenden Probe gemessenen VWF-Aktivitäten [% der Norm] bei zusätzlicher Verwendung verschiedener Verdünnungen des kommerziellen HBR-1-Reagenzes (mg/mL Gesamtproteingehalt im Reaktionsgemisch) (1), bei zusätzlicher Verwendung verschiedener Konzentrationen der neuen Antikörpervariante im Reaktionsgemisch (mg/mL) (2) beziehungsweise bei zusätzlicher Verwendung verschiedener Konzentrationen der neuen Antikörpervariante im Reaktionsgemisch (mg/mL) in Kombination mit dem HBR-1 Reagenz (3) dargestellt sind.

### BEISPIELE

### BEISPIEL 1: Latexagglutinationsassay zur Bestimmung der VWF-Aktivität gemäß dem Stand der Technik

### Reagenz 1:

HBR-1 Reagenz (Heterophilic Blocking Reagent 1, Scantibodies Laboratory, Inc., Santee, USA) enthaltend eine Mischung muriner Immunglobuline zur Bindung an heterophile Antikörper

### Reagenz 2:

Rekombinant exprimiertes GPIb-Proteinfragment einer gain-offunction Mutante des humanen GPIb-Proteins in Pufferlösung.

### Reagenz 3:

Suspension von mit einem murinen, monoklonalen anti-GPIb-Antikörper beschichteten Polystyrenpartikeln (Latexpartikeln).

Zu Bestimmung der von Willebrand Faktor (VWF)-Aktivität in einer Plasmaprobe wird wie folgt verfahren:
1. 40 µL Probe werden mit 2 µL Reagenz 1 vermischt, und die so behandelte Probe wird für 30 Minuten bei Raumtemperatur inkubiert.
2. Anschließend werden 15 µL der vorbehandelten Probe mit 30 µL Owren's Veronal Puffer und mit 70 µL eines weiteren, detergenzhaltigen Puffers und mit 15 µL des Reagenz 2 vermischt, und das Gemisch wird für 2 Minuten bei 37 °C inkubiert.
3. Anschließend werden dem Gemisch 40 µL Reagenz 3 zugegeben, und die Extinktionsänderung des Reaktionsgemisches wird mit Licht einer Wellenlänge von 570 nm gemessen.
4. Die gemessenen Rohwerte werden mithilfe eine Kalibrationskurve ausgewertet.

Trotz Verwendung des HBR-1 Reagenzes gibt es gelegentlich Proben, für die falsch zu hohe Ergebnisse ermittelt werden, weil die durch das Reagenz 1 beabsichtigte HAMA-Blockierung offenbar nicht wirksam genug ist.

### BEISPIEL 2: Herstellung einer Variante des anti-GPIb-Antikörpers mit verminderter GPIb-Bindungsfähigkeit

Die vollständige Aminosäuresequenz des in dem VWF-Assay gemäß Beispiel 1 als analytischen Antikörper eingesetzten murinen, monoklonalen anti-GPIb-Antikörpers wurde ermittelt.

Die Aminosäuresequenz im Bereich der schweren, variablen Kette dieses anti-GPIb-Antikörpers umfassend die komplementaritätsbestimmenden Regionen CDR-H1, CDR-H2 und CDR-H3 (gemäß Kabat-Nummerierungsschema) lautete wie folgt:

QVQLKESGPGLVAPSQSLSI TCTVSGFSLTDYGVNWVRQP
1 20 21 40

PGKGLEWLGMIWVDGSTDYN SALKSRLSISKDNSKSQVFL
41 60 61 80

KMNSLQTDDTARYYCARDTM IKGHYVMDYWGQGTSVTVSS
81 100 101 120

(SEQ ID NO. 3)

Zur Herstellung einer Variante dieses anti-GPIb-Antikörpers mit verminderter GPIb-Bindungsfähigkeit wurden in der CDR-H3-Region die Aminosäurereste an den Positionen 105 und 107 der SEQ ID NO. 3 ausgetauscht. An Position 105 wurde die relativ große Aminosäure Tyrosin (Y) durch die sehr kleine und kurze Aminosäure Serin (S) ersetzt (in beiden Sequenzdarstellungen fettgedruckt). An Position 107 wurde Methionin (M) durch Phenylalanin (F) ersetzt, was einer Rückkehr zur Germline des Mausantikörpers entspricht (in beiden Sequenzdarstellungen fettgedruckt). Es wurde ein entsprechend codierendes Nukleinsäuremolekül abgeleitet und mit gentechnischen Standardmethoden eine transgene Expressionszelllinie etabliert, die den modifizierten Antikörper exprimiert, dessen Aminosäuresequenz mit Ausnahme der beiden genannten veränderten Aminosäurereste identisch ist mit der Aminosäuresequenz des anti-GPIb-Antikörpers.

Die Aminosäuresequenz im Bereich der schweren, variablen Kette dieses modifizierten Antikörpers umfassend die komplementaritätsbestimmenden Regionen CDR-H1, CDR-H2 und CDR-H3 lautete also wie folgt:

QVQLKESGPGLVAPSQSLSI    TCTVSGFSLTDYGVNWVRQP
1                  20    21                  40

PGKGLEWLGMIWVDGSTDYN    SALKSRLSISKDNSKSQVFL
41                 60    61                  80

KMNSLQTDDTARYYCARDTM    IKGHSVFDYWGQGTSVTVSS
81                100    101                120

(SEQ ID NO. 4)

### BEISPIEL 3: Nachweis der verminderten GPIb-Bindungsfähigkeit der neuen Antikörpervariante

Als Testsystem wurde der Latexagglutinationsassay zur Bestimmung der VWF-Aktivität gemäß Beispiel 1 verwendet.

Der Assay wurde dahingehend modifiziert, dass anstelle des Reagenzes 1 (HBR-1 Reagenz) jeweils 2 µL von Reagenzien, die unterschiedliche Mengen der gemäß Beispiel 2 hergestellten neuen Antikörpervariante enthielten, mit 40 µL einer normalen Plasmaprobe beziehungsweise einer Probe mit bekanntermaßen verminderter VWF-Aktivität vermischt und inkubiert wurden.

Die Ergebnisse sind in FIG. 1A dargestellt. Bei Endkonzentrationen von bis zu 0,02 mg/mL der neuen Antikörpervariante im finalen Reaktionsgemisch verringert sich die gemessene VWF-Aktivität im Vergleich zu Reaktionsgemischen, denen die neue Antikörpervariante nicht zugegeben wurde (0 mg/mL), um maximal 2,3 %. Dies zeigt, dass die neue Antikörpervariante nicht mit dem funktionellen ("analytischen") murinen, monoklonalen anti-GPIb-Antikörper um die Bindungsstelle am GPIb-Protein konkurriert.

Die gemäß Beispiel 2 hergestellte neuen Antikörpervariante, deren Aminosäuresequenz mit Ausnahme der beiden genannten veränderten Aminosäurereste identisch ist mit der Aminosäuresequenz des funktionellen anti-GPIb-Antikörpers, verfügt demnach über eine gegenüber dem funktionellen anti-GPIb-Antikörper stark verminderte GPIb-Bindungsfähigkeit.

Zu Vergleichszwecken wurde der Assay in einer weiteren Variante dahingehend modifiziert, dass jeweils 2 µL unterschiedlicher Verdünnungen des Reagenzes 1 (HBR-1 Reagenz), die unterschiedliche Gesamtproteinkonzentrationen enthielten, mit 40 µL einer normalen Plasmaprobe beziehungsweise einer Probe mit bekanntermaßen verminderter VWF-Aktivität vermischt und inkubiert wurden.

Die Ergebnisse sind in FIG. 1B dargestellt. Bei Endkonzentrationen von bis zu 0,02 mg/mL HBR-Gesamtprotein im finalen Reaktionsgemisch verringert sich die gemessene VWF-Aktivität im Vergleich zu Reaktionsgemischen, denen kein HBR-1 Reagenz zugegeben wurde (0 mg/mL), um maximal 2,6 %. Diese Beobachtung stützt die Schlussfolgerung, dass es sich bei der durch die neue Antikörpervariante verursachten Verringerung der VWF-Aktivität nicht um einen spezifischen Effekt der Antikörpervariante handelt.

### BEISPIEL 4: Nachweis der HAMA-Antikörper blockierenden Wirkung der neuen Antikörpervariante

Der Latexagglutinationsssay zur Bestimmung der VWF-Aktivität gemäß Beispiel 1 wurde dahingehend modifiziert, dass anstelle des Reagenzes 1 (HBR-1 Reagenz) jeweils 2 µL von Reagenzien, die unterschiedliche Mengen der gemäß Beispiel 2 hergestellten neuen Antikörpervariante alleine beziehungsweise unterschiedliche Mengen der gemäß Beispiel 2 hergestellten neuen Antikörpervariante in Kombination mit dem Reagenz 1 (HBR-1 Reagenz) enthielten, mit 40 µL einer HAMA-Antikörper-enthaltenden Plasmaprobe mit bekannter VWF-Aktivität vermischt und inkubiert wurden. Die verwendete Probe zeichnete sich dadurch aus, dass sie durch die alleinige Verwendung von HBR-1 Reagenz nicht ausreichend geblockt werden konnte und deshalb eine falsch zu hohe VWF-Aktivität bestimmt wurde.

Die Ergebnisse sind in FIG. 2 dargestellt. Bei Endkonzentrationen ab 0,005 mg/mL der neuen Antikörpervariante im finalen Reaktionsgemisch ist bereits eine nahezu vollständige Blockade der interferierenden Wirkung der HAMA-Antikörper zu beobachten. Die alleinige Verwendung von HBR-1 Reagenz bewirkt hingegen nur eine unzureichende Blockade der interferierenden Wirkung der HAMA-Antikörper. Die Kombination der neuen Antikörpervariante mit dem HBR-1 Reagenz zeigte keine Blockierungswirkung, die über die Blockierungswirkung der neuen Antikörpervariante hinausging oder die auf eine Beeinträchtigung hinwies.

## Patentansprüche

1. Kit zur Verwendung in einem Verfahren zum Nachweis eines Analyten in einer Körperflüssigkeitsprobe, das Kit enthaltend
a) einen ersten, Antigen-spezifischen Antikörper mit einer ersten Aminosäuresequenz, der spezifisch an ein Antigen bindet und dessen Verwendung in einem definierten Testsystem zum Nachweis des Analyten eine Analyt-spezifische Nachweisreaktion bewirkt, und
b) eine Antikörpervariante mit einer zweiten Aminosäuresequenz, deren Antigen-Bindungsfähigkeit im Vergleich zu dem ersten Antikörper so stark vermindert ist, dass deren zusätzliche Verwendung in dem definierten Testsystem zum Nachweis des Analyten die Analyt-spezifische Nachweisreaktion um maximal 15 % verringert,
**dadurch gekennzeichnet, dass**
die Aminosäuresequenz der Antikörpervariante mit Ausnahme von ein bis drei veränderten Aminosäureresten identisch ist mit der Aminosäuresequenz des ersten Antigen-spezifischen Antikörpers.

2. Kit gemäß Anspruch 1, wobei die ein bis drei veränderten Aminosäurereste in einer oder mehreren der komplementaritätsbestimmenden Regionen (CDR's) der schweren oder der leichten Kette der Antikörpervariante lokalisiert sind.

3. Kit gemäß Anspruch 2, wobei mindestens ein veränderter Aminosäurerest in einer komplementaritätsbestimmenden Region der schweren Kette der Antikörpervariante lokalisiert ist.

4. Kit gemäß Anspruch 3, wobei mindestens ein veränderter Aminosäurerest in der komplementaritätsbestimmenden Region CDR-H3 der schweren Kette der Antikörpervariante lokalisiert ist.

5. Kit gemäß einem der vorhergehenden Ansprüche, wobei mindestens die Antikörpervariante rekombinant hergestellt wurde.

6. Kit gemäß einem der vorhergehenden Ansprüche, wobei der erste, Antigen-spezifische Antikörper und die Antikörpervariante in unterschiedlichen Reagenzien enthalten sind.

7. Kit gemäß einem der vorhergehenden Ansprüche, wobei der erste, Antigen-spezifische Antikörper mit einer Festphase und/oder einer Komponente eines signalgebenden Systems assoziiert ist.

8. Kit gemäß einem der vorhergehenden Ansprüche, wobei der erste, Antigen-spezifische Antikörper ein Analyt-spezifischer Antikörper ist.

9. Kit gemäß einem der vorhergehenden Ansprüche, ferner enthaltend
c) mindestens einen weiteren Antigen-spezifischen Antikörper mit einer dritten Aminosäuresequenz der spezifisch an ein Antigen bindet und dessen Verwendung in dem definierten Testsystem zum Nachweis des Analyten eine Analyt-spezifische Nachweisreaktion bewirkt und
d) eine weitere Antikörpervariante mit einer vierten Aminosäuresequenz, deren Antigen-Bindungsfähigkeit im Vergleich zu dem weiteren Antigen-spezifischen Antikörper so stark vermindert ist, dass deren zusätzliche Verwendung in dem definierten Testsystem zum Nachweis des Analyten die Analyt-spezifische Nachweisreaktion um maximal 15 % verringert,
und wobei die Aminosäuresequenz der weiteren Antikörpervariante mit Ausnahme von ein bis drei veränderten Aminosäureresten identisch ist mit der Aminosäuresequenz des weiteren Antigen-spezifischen Antikörpers.

10. Kit gemäß Anspruch 1, wobei der erste, Antigen-spezifische Antikörper spezifisch an GPIb-Protein bindet.

11. Kit gemäß Anspruch 10, wobei der erste, GPIb-Proteinspezifische Antikörper in der komplementaritätsbestimmenden Region CDR-H3 der schweren Kette die Aminosäuresequenz gemäß SEQ ID NO. 1 aufweist.

12. Kit gemäß Anspruch 11, wobei die Aminosäuresequenz der Antikörpervariante mit Ausnahme von zwei veränderten Aminosäureresten identisch ist mit der Aminosäuresequenz des ersten GPIb-Protein-spezifischen Antikörpers und in der komplementaritätsbestimmenden Region CDR-H3 der schweren Kette die Aminosäuresequenz gemäß SEQ ID NO. 2 aufweist.

13. Verwendung eines Kits gemäß einem der vorhergehenden Ansprüche in einem Verfahren zum Nachweis eines Analyten in einer Körperflüssigkeitsprobe.

14. Verwendung eines Kits gemäß einem der Ansprüche 1 bis 15 zum interferenzfreien Nachweis eines Analyten in einer Körperflüssigkeitsprobe, die interferierende Antikörper, beispielsweise aus der Gruppe heterophile Antikörper und Autoantikörper, enthält.

15. Verwendung eines Kits gemäß einem der Ansprüche 10 bis 12 in einem Verfahren zum Nachweis der VWF-Aktivität in einer Körperflüssigkeitsprobe.

16. Verfahren zum Nachweis eines Analyten in einer Körperflüssigkeitsprobe, das Verfahren umfassend die Schritte:
a) Bereitstellen eines Reaktionsgemisches durch Vermischen der Probe mit
i. einem ersten, Antigen-spezifischen Antikörper mit einer ersten Aminosäuresequenz, der spezifisch an ein Antigen bindet und dessen Verwendung in einem definierten Testsystem zum Nachweis des Analyten eine Analyt-spezifische Nachweisreaktion bewirkt, und
ii. einer Antikörpervariante mit einer zweiten Aminosäuresequenz, deren Antigen-Bindungsfähigkeit im Vergleich zu dem ersten Antikörper so stark vermindert ist, dass deren zusätzliche Verwendung in dem definierten Testsystem zum Nachweis des Analyten die Analyt-spezifische Nachweisreaktion um maximal 15 % verringert; und
b) Messen einer Messgröße in dem Reaktionsgemisch, wobei die Messgröße von der Bildung eines Komplexes aus Antigen und dem ersten, Antigen-spezifischen Antikörper beeinflusst wird, und die mit der Menge des Analyten korreliert,
**dadurch gekennzeichnet, dass**
die Aminosäuresequenz der Antikörpervariante mit Ausnahme von ein bis drei veränderten Aminosäureresten identisch ist mit der Aminosäuresequenz des ersten, Antigen-spezifischen Antikörpers.

17. Verfahren gemäß Anspruch 16, wobei die Probe zuerst mit der Antikörpervariante vermischt wird, das so entstandene Gemisch inkubiert wird und dann dem Gemisch der erste, Antigen-spezifische Antikörper zugegeben wird.

18. Verfahren gemäß einem der Ansprüche 16 und 17, wobei der erste, Antigen-spezifische Antikörper ein Analyt-spezifischer Antikörper ist und wobei die gemessene Messgröße von der Bildung eines Komplexes aus Analyt und dem ersten, Analyt-spezifischen Antikörper beeinflusst wird.

19. Verfahren gemäß einem der Ansprüche 16 und 17, wobei der erste, Antigen-spezifische Antikörper ein Antikörper mit Spezifität für einen Bindungspartner des Analyten ist, und wobei die gemessene Messgröße von der Bildung eines Komplexes aus Analyt und dem Bindungspartner des Analyten und dem ersten, Antigen-spezifischen Antikörper mit Spezifität für den Bindungspartner des Analyten beeinflusst wird.

20. Verfahren gemäß Anspruch 19 zum Nachweis der Aktivität des von Willebrand Faktors in einer Körperflüssigkeitsprobe, wobei der erste, Antigen-spezifische Antikörper ein Antikörper mit Spezifität für GPIb-Protein ist, und wobei die gemessene Messgröße von der Bildung eines Komplexes aus von Willebrand Faktor und dem GPIb-Protein und dem ersten, Antigen-spezifischen Antikörper mit Spezifität für GPIb-Protein beeinflusst wird.

21. Verfahren gemäß einem der Ansprüche 16 bis 20, wobei der erste, Antigen-spezifische Antikörper mit einer partikulären Festphase assoziiert ist und die Agglutination der partikulären Festphase, die von der Bildung eines Komplexes aus Antigen und dem ersten, Antigen-spezifischen Antikörper beeinflusst wird und die mit der Menge des Analyten korreliert, im Reaktionsgemisch gemessen wird.
